# EUROPEAN PATENT SPECIFICATION

(11) **EP 0 619 817 B1**
(45) Date of publication and mention of the grant of the patent: **17.03.1999**
(21) Application number: 93921055.5
(22) Date of filing: 08.09.1993
(51) Int. Cl.: C07D 311/60, A61K 31/35

(54) **NOVEL PHARMACEUTICALLY ACTIVE FLAVILIUM COMPOUNDS**
PHARMAZEUTISCH AKTIVE FLAVILIUM VERBINDUNGEN
NOUVEAUX COMPOSES DE FLAVILIUM ACTIFS SUR LE PLAN PHARMACEUTIQUE

(30) Priority: 21.09.1992 HU 300092
(43) Date of publication of application: 19.10.1994
(73) Proprietor: BIOGAL GYOGYSZERGYAR RT., 4042 Debrecen (HU)
(72) Inventor: SULYOK, György, H-4027 Debrecen (HU); BALINT, János, H-4032 Debrecen (HU); BORBELY, Ildikú, H-4032 Debrecen (HU); KISS, Jolán, H-4029 Debrecen (HU); D. T TH, Ferenc, H-4028 Debrecen (HU)
(74) Representative: Hansen, Bernd, Dr. Dipl.-Chem.
(86) International application number: PCT/HU93/00052
(87) International publication number: WO 94/06787

(56) References cited:
- CH-A- 639 557
- DE-C- 2 808 823

## Description

The present invention relates to novel flavilium derivatives of general formula (I)
wherein
- X: is a non-toxic anion,
- R₁ and R₂: are independently selected from hydrogen or -OY group in which
Y is hydrogen, α-amino-acyl group or amino-alkyl group of 1 to 5 carbon atoms;
- R₅: is hydrogen, α-amino-acyl group or amino-alkyl group of 1 to 5 carbon atoms;
- R₃, R₄, and R₇: independently are hydrogen or alkyl group of 1 to 5 carbon atoms,
- R₆: is hydrogen, hydroxy group or alkyl group of 1 to 5 carbon atoms.

In the general formula (I) Y, R₃, R₄, R₅ R₆ and R₇ can be an alkyl group of 1 to 5 carbon atoms e.g. methyl, ethyl, propyl, isopropyl, n-butyl, sec-butyl terc-butyl, n-pentyl or isopentyl group, preferably methyl group; the Á-amino-acyl group can be the acyl radical of an essential amino acid, preferably glicyl, alanyl, lysyl or arginyl group.

The compounds of general formula (I) possess outstanding selective anti-viral effect. Their effect against HIV virus is particularly important; comparing them to AZT they proved to be more effective and less toxic.

The flavanoids are well-known wide-spread colour substances in the flora. Flavilium salts form a type of this family of compounds. These salts are widely used in food industry e.g. for colouring fruits and other vegetable foods (e.g. German Patent No. 1,904,810, GDR Patent No. 140,353, US Patent No. 3,314,975).

Medical application of flavilium derivatives, their lipid and cholesterin level decreasing effect is described in European Patent No. 19,524, German Patent No. 2,808,820, while the effect of flavilium chloride similar to that of vitamin P is described in German Patents No. 2,002,421 and No. 2,703,375.

Flavilium derivatives can be obtained from plant by various extraction methods or by synthetic procedures. According to one of synthetic methods a suitable phenol derivative is condensed with a substituted chalcone or phenyl-β-diketon e.g. pursuant to German Patent No. 1,904,810 and No. 2,808,823 or α-chloro-β-N,N-dimethylformimidoyl-styrene perchlorate is used as a condensation partner in order to produce 7-amino-flavilium salts, in accordance with GDR Patent No. 140,353.

According to the other possible way of reaction the desired compounds are prepared from a natural vegetable basic material by various reucing methods (Mg + sulfuric acid, LiAlH₄, Zn + acetic anhydride) (German Patents No. 2,002,421 and No. 2,703,375, European Patent No. 19,524 and Japanese Patent No. 55,036,679).

According to our experience preparation of compounds of general formula (I) can not be realized by the above methods partly because there is no suitable vegetable basic material and because the methylated coumarin derivative and methylated phenol starting materials practically do not go into condensation reaction under normal conditions.

It is well known that according to the Goswami-Chakravarti synthesis coumarin is condensed with resorcine in such a way that the reactants are heated in the presence of phosphorus oxychloride of catalytic amount (J. Ind. Chem. Soc. 9, 599, 1932).

The present invention provides compounds of the formula (I): 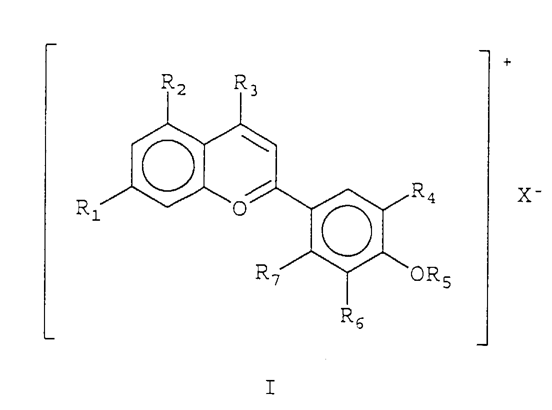 wherein:
X is a non-toxic anion;
R₁ and R₂ are independently selected from hydrogen or an -OY group in which Y is hydrogen, an α-amino-acyl group or an amino-alkyl group having 1 to 5 carbon atoms;
R₅ is hydrogen, an α-amino-acyl group or an amino-alkyl group having 1 to 5 carbon atoms;
R₃, R₄, and R₇ independently are hydrogen or an alkyl group having 1 to 5 carbon atoms; and
R₆ is hydrogen, a hydroxy group or an alkyl group having 1 to 5 carbon atoms.

We found that the compounds of general formula (I) can be prepared by analogy to Goswami-Chakravarti synthesis in such a way that a phenol derivative of general formuia (II), wherein
- R₄, and R₇: are hydrogen or alkyl group of 1 to 5 carbon atoms;
- R₈: is hydrogen
- R₆: is hydrogen, hydroxy or alkyl group of 1 to 5 carbon atoms
is condensed with a coumarin derivative of general formula (III), wherein
- R₁ and R₂: are hydrogen or hydroxy group;
- R₃: is hydrogen or alkyl group of 1 to 5 carbon atoms.

Condensation is performed in the presence of a much higher amount of phosphorus oxychloride than the amount used in the above-mentioned reactions.

It is preferable to use 1.0 to 1.5 moles of phosphorus oxychloride calculated for the reactants. Instead of phosphorus oxychloride Lewis acids e.g. zinc chloride can also be used. Condensation takes place at a temperature in the range 70 to 115°C in the course of reaction.

In order to obtain a better yield it is preferable to use hydrochloric acid gas. In that case the reaction takes place at room temperature, as well.

Alternatively, compounds of general formula (I) can be obtained by condensing a phenol of the general formula (II), wherein
- R₄ , and R₇: are hydrogen or alkyl group of 1 to 5 carbon toms;
- R₈: is acetoacetyl group;
- R₆: is hydrogen, hydroxy or alkyl group of 1 to 5 carbon atoms
with a compound of general formula (IV), wherein R₁ and R₂ are hydrogen or hydroxy group.

Preferably the condensation is performed at a temperature between 15 and 30°C in the presence of hydrochlric acid gas.

In both of the above procedures the hydrochloride salt is separated and in optional cases another pharmaceutically accepted salt is prepared by a ususal method.

If desired, the compound of general formula (I) wherein Y and/or R₅ are hydrogen is
i/ transformed into an ester with an α-amino-acid, or
ii/ reacted with an amino-alkyl-halide of 1 to 5 carbon atoms.

Esterification can be carried out e.g. by coupling the suitable compound of formula (I) with an N-protected α-amino-acid by means of a coupling reagent such as dicyclohexilcarbodiimide. The amino protecting group can be a usual protecting group e.g. tertiary butoxy-carbonyl group is to be removed by acidic treatment after the coupling reaction.

In order to examine the anti-HIV effect of compounds of general formula I the reverse transcriptase activity was measured ( Hoffman, A. D. et al. Virology 147, 326-335 1985).

Our results are demonstrated by means of the model compound 5,7,4'-trihydroxy-4,3',5'-trimethyl-flavilium chloride (BIOGAL 6) obtained according to Example 1.

Cell and virus. To test the active agents we used MT-4 cell-line infected with strain HIV-1 IIIB. The cells were infected with 20 TCID₅₀ HIV-1 virus.

The testing procedure. The following concnetrations of the active agents were tested of 5 µg/cm³, 20 µg/cm³ and 100 µg/cm³. The active agent was added to the culture first 4 hours before the infection, than 24 and 48 hours after infection. We used HIV infected, untreated MT-4 cells and uninfected, untreated cells as a control. Retrovir we used for the azidothimidin (AZT)-control was purchased from firm Wellcome.

Study of the reverz transcriptase activity. We took 500 µl aliquots from supernatant of the culture and measured their virus concentration. The result are given in cpm/cm³ which indicates ³H-TTP.

### Results.

Table 1 shows the activity level of revers transcriptase indicating the effect on virus production. The activity was measured every 3-4 days, but Table 1 shows only the represesentative results from the 7th, 10th, and 18th days.

**Table 1**

| Effect of the BIOGAL 6 and AZT on the HIV virus propagation | | | | |
|---|---|---|---|---|
| Active substance | Dose µg/cm³ | RT activity (cpm/cm³) | | |
| | | 7. day | 10 day | 18 day |
| | 5 | 1.795 | 1.228 | 1.198 |
| BIOGAL 6 | 20 | 1.748 | 1.626 | 1.300 |
| | 100 | 1.666 | 1.300 | 1.359 |
| | 5 | 2.195 | 2.127 | 38.797 |
| AZT | 20 | 2.386 | 2.224 | 1.921 |
| | 100 | 2.220 | 2.181 | 1.838 |
| HIV CONTROL | - | 113.000 | 84.000 | 28.211 |

As one can see BIOGAL-6 compound completely blocked the HIV production at 5 µg/cm³ concentration. AZT also suppressed HIV proliferation at 5 µg/cm³ concentration but this effect was shorter than the effect of BIOGAL-6 compound.

### Comparison of effects of BIOGAL-6 compound and AZT

The effects of both compounds were tested at the following concentrations: 5.0, 2.5, 1.0, 0.5, 0.1 µg/cm³. Table 2 summarizes representative results. One can see that BIOGAL-6 compound was completely ineffective at 1.0 and less 1.0 µg/cm³ concentrations. However the effect of BIOGAL-6 compound at 5.0 and 2.5 µg/cm³ concentrations was long-lasting, while administration of AZT had temporary effect even in higher concentrations.

**Table 2**

| Comparison of effects of BIOGAL-6 and AZT | | | | |
|---|---|---|---|---|
| Active substance | Dose µg/cm³ | RT activity (cpm/cm³) | | |
| | | 7. day | 10 day | 18 day |
| | 5.0 | 1.478 | 1.019 | 1.226 |
| | 2.5 | 6.231 | 8.938 | 6.803 |
| BIOGAL-6 | 1.0 | 96.638 | 75.917 | 38.595 |
| | 0.5 | 108.846 | 98.150 | 43.804 |
| | 0.1 | 111.035 | 99.813 | 45.535 |
| | 5.0 | 1.724 | 3.571 | 51.735 |
| | 2.5 | 3.231 | 9.083 | 116.001 |
| AZT | 1.0 | 3.989 | 15.115 | 75.539 |
| | 0.5 | 6.148 | 75.051 | 84.047 |
| | 0.1 | 7.392 | 125.627 | 62.817 |
| HIV control | - | 122.970 | 95.047 | 59.260 |

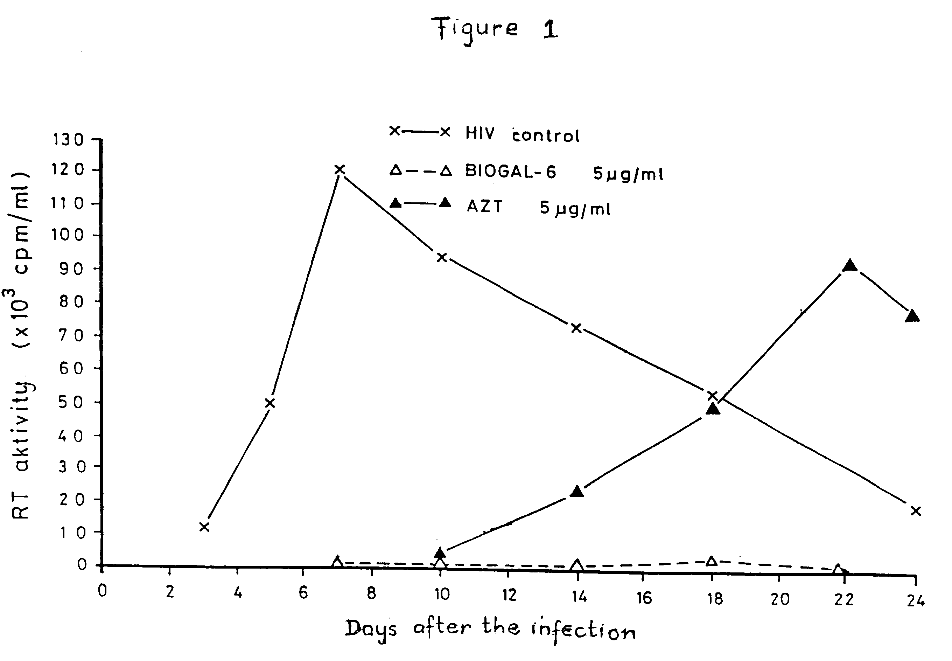

Fig. 1 shows the kinetics of the effect of BIOGAL-6 compound. As the Figure 1 shows BIOGAL-6 compound completely blocked HIV production at 5µg/cm³ concentration, while AZT at the same concentration only delayed the HIV production but did not change the proliferation rate. The effect of BIOGAL-6 compound compared to AZT was long-lasting. This difference suggests that the two compounds exert their action through different mechanisms.

According to the acut toxitity examinations (CFLP/SPF mice; per os and i. v.) the BIOGAL-6 compound is not toxic. The invention is illustrating in detail by aid of the following non-limiting Examples.

### Example 1

Preparation of 5,7,4'-trihydroxy-4,3',5'-trimethyl-flavilium chloride:
4-methyl-5,7-dihydroxy-coumarin (3.842 g) and 2,6-dimethylphenol (2,44 g) are kept boiling with total reflux in the presence of 10 cm³ of POCl₃ ans 3 g of ZnCl₂ at 100°C for 30 minutes then the mixture is poured into 200 cm³ of icy water. The precipitate is dissolved in 100 cm³ of methanol. The methanol solution is treated with 3 dm³ of ethyl acetate. The precipitate is filtered off, dissolved in methanil and chromatografed on Fractogel PGM 2000 adsorbent. The eluted material is recovered under reduced pressure at 60°C in a rotary waporater. The ethyl acetate separation and the chromatographic purification steps are repeated if necessary.
Identification data of the product:
R_{f}: 0.58 Merck Cellulose (5632) water: hydrochloric acid: acetic acid = 3:2:5 v/v
¹H NMR (200 MH; DMSO_{d6}:
2.25 ppm: 2 CH₃; 2.96 ppm: 1 CH₃; 6.8-6.925 ppm: H6, H8; 8.0 ppm: H2', H6'; 8,025 ppm: H3

### Example 2

Preparation of 5,7,4'-trihydroxy-4,3',5'-trimethyl-flavilium chloride:
4 g of 2,6-dimethyl-4-acetoacetyl-phenol and 4 g of phloroglucinol are dissolved in 80 ml of water-free ethyl alcohol then hydrochloric acid gas is introduced and the solution is stirred at room temperature. Progress of the reaction is followed by thin layer chromatography. When the ring-closure is finished (appr. 8 ours) the reaction mixture is poured into water, the precipitate is filtered off and purified by column chromatography.
Yield: 4,23 g
Identification data of the product are the same as those described in Example 1.

### Example 3

Preparation of 4'5,7-trihydroxy-4,3',5'-trimethyl flavilium chloride-5,7-dialanyl ester
1.3 g pf the compound prepared according to Example 1 is reacted with 1.5 g of N-BOC-alanine (BOC = terc butoxy-carbonyl group) in the presence of 1.6 g of dicyclohexylcarbodiimide. At the end of the reaction the precipitated diciclohexyl-urea is filtered off, the solvent is removed from the filtrate and the residue is dissolved in ethyl acetate. The solution is evaporated again and the title product is obtained after removing the protecting group by acidic treatment.
Yield: 1.6 g

## Claims

1. Compounds of the formula (I): 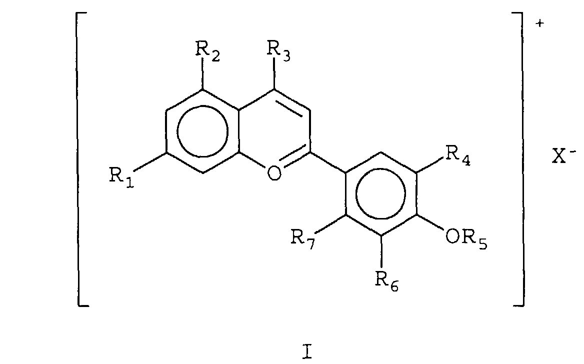 wherein:
X is a non-toxic anion;
R₁ and R₂ are independently selected from hydrogen or an -OY group in which Y is hydrogen, an α-amino-acyl group or an amino-alkyl group having 1 to 5 carbon atoms;
R₅ is hydrogen, an α-amino-acyl group or an amino-alkyl group having 1 to 5 carbon atoms;
R₃, R₄, and R₇ independently are hydrogen or an alkyl group having 1 to 5 carbon atoms; and
R₆ is hydrogen, a hydroxy group or an alkyl group having 1 to 5 carbon atoms.

2. A compound according to Claim 1, wherein:
X is a chloride ion;
R₁ and R₂ are each an -OY group in which Y is an amino-alkyl group having 1 to 5 carbon atoms or a glycyl, an alanyl, a lysyl or an arginyl group;
R₅ is hydrogen;
R₃, R₄, and R₇ are a hydrogen or a methyl group; and
R₆ is hydrogen, a hydroxy group or a methyl group.

3. A compound according to Claim 1, which is 5,7,4'-trihydroxy-4,3',5'-trimetil-flavilium chloride.

4. A process for the preparation of compounds of the formula (I): 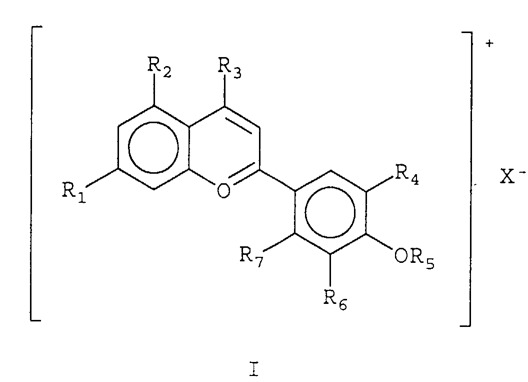 wherein:
X is a non-toxic anion;
R₁ and R₂ are independently selected from hydrogen or an -OY group in which Y is hydrogen, an α-amino-acyl group or an amino-alkyl group having 1 to 5 carbon atoms;
R₅ is hydrogen, an α-amino-acyl group or an amino-alkyl group having 1 to 5 carbon atoms;
R₃, R₄, and R₇ are independently hydrogen or an alkyl group having 1 to 5 carbon atoms; and
R₆ is hydrogen, a hydroxy group or an alkyl group having 1 to 5 carbon atoms,
which process is characterised in that either:
a) a phenol derivative of the formula (II): 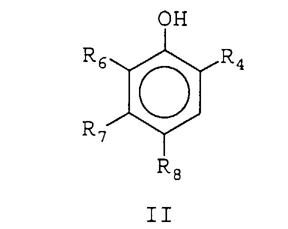 wherein:
R₄, and R₇ are hydrogen or an alkyl group having 1 to 5 carbon atoms;
R₆ is hydrogen, or a hydroxy or alkyl group having 1 to 5 carbon atoms; and
R₈ is hydrogen,
is condensed with a coumarin derivative of the formula (III): 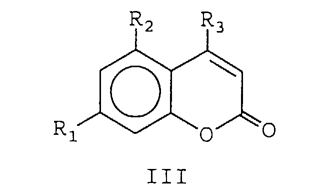 wherein:
R₁ and R₂ are hydrogen or a hydroxy group;
R₃ is hydrogen or an alkyl group having 1 to 5 carbon atoms;
or
b) a phenol derivative of formula (II), wherein:
R₄, and R₇ are hydrogen or an alkyl group having 1 to 5 carbon atoms;
R₆ is hydrogen, or a hydroxy or alkyl group having 1 to 5 carbon atoms; and
R₈ is an acetoacetyl group;
is condensed with a compound of the formula (IV), 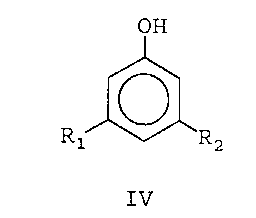 wherein:
R₁ and R₂ are hydrogen or a hydroxy group;
in the presence of phosphorus oxychloride or hydrochloric acid gas, and, if desired, when Y and/or R₅ are hydrogen, the compound of formula (I) is:
(i) transformed into an ester with an α-amino-acid, or
(ii) reacted with an amino-alkyl-halide having 1 to 5 carbon atoms.

5. A compound as defined in any of Claims 1 to 3 for use as a pharmaceutical.

6. A pharmaceutical composition comprising a compound as defined in any of Claims 1-3 and a pharmaceutically acceptable carrier.

7. Use of a compound as defined in any of Claims 1-3 for the manufacture of a medicament effective against HIV.

## Patentansprüche

1. Verbindungen der Formel (I): 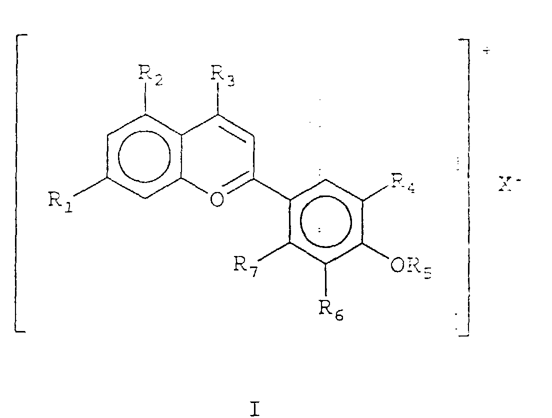 worin
X ein untoxisches Anion ist,
R₁ und R₂ unabhängig voneinander aus Wasserstoff oder einer -OY-Gruppe ausgewählt sind, worin Y Wasserstoff, eine α-Aminoacylgruppe oder eine Aminoalkylgruppe mit 1 bis 5 Kohlenstoffatomen bedeutet;
R₅ Wasserstoff, eine α-Aminoacylgruppe oder eine Aminoalkylgruppe mit 1 bis 5 Kohlenstoffatomen;
R₃, R₄ und R₇ unabhängig voneinander Wasserstoff oder eine Alkylgruppe mit 1 bis 5 Kohlenstoffatomen; und
R₆ Wasserstoff, eine Hydroxylgruppe oder eine Alkylgruppe mit 1 bis 5 Kohlenstoffatomen bedeuten.

2. Verbindung gemäß Anspruch 1, worin
X ein Chloridion ist;
R₁ und R₂ jedes eine -OY-Gruppe ist, worin Y eine Aminoalkylgruppe mit 1 bis 5 Kohlenstoffatomen oder eine Glycyl-, Alanyl-, Lysyl- oder Arginylgruppe bedeutet;
R₅ Wasserstoff;
R₃, R₄ und R₇ Wasserstoff oder eine Methylgruppe; und
R₆ Wasserstoff, eine Hydroxylgruppe oder eine Methylgruppe bedeuten.

3. Verbindung gemäß Anspruch 1, die 5,7,4'-Trihydroxy-4,3',5'-trimethyl-flaviliumchlorid ist.

4. Verfahren zur Herstellung von Verbindungen der Formel (I): 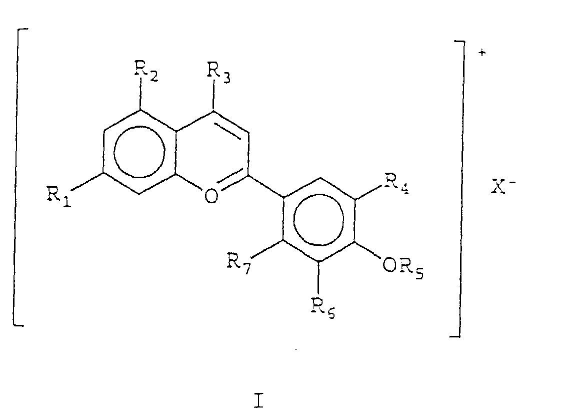 worin
X ein untoxisches Anion ist,
R₁ und R₂ unabhängig voneinander aus Wasserstoff oder einer -OY-Gruppe ausgewählt sind, worin Y Wasserstoff, eine α-Aminoacylgruppe oder eine Aminoalkylgruppe mit 1 bis 5 Kohlenstoffatomen bedeutet;
R₅ Wasserstoff, eine α-Aminoacylgruppe oder eine Aminoalkylgruppe mit 1 bis 5 Kohlenstoffatomen;
R₃, R₄ und R₇ unabhängig voneinander Wasserstoff oder eine Alkylgruppe mit 1 bis 5 Kohlenstoffatomen; und
R₆ Wasserstoff, eine Hydroxylgruppe oder eine Alkylgruppe mit 1 bis 5 Kohlenstoffatomen bedeuten,
welches Verfahren dadurch gekennzeichnet ist, daß entweder
a) ein Phenolderivat der Formel (II) 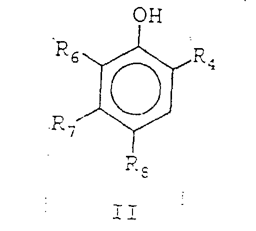 worin R₄ und R₇ Wasserstoff oder eine Alkylgruppe mit 1 bis 5 Kohlenstoffatomen;
R₆ Wasserstoff, eine Hydroxylgruppe oder eine Alkylgruppe mit 1 bis 5 Kohlenstoffatomen; und
R₈ Wasserstoff bedeuten,
mit einem Cumarinderivat der allgemeinen Formel (III): 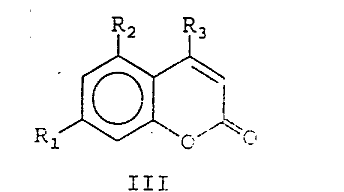 worin R₁ und R₂ Wasserstoff oder eine Hydroxylgruppe und
R₃ Wasserstoff oder eine Alkylgruppe mit 1 bis 5 Kohlenstoffatomen bedeuten, kondensiert wird, oder
b) ein Phenolderivat der Formel (II), worin R₄ und R₇ Wasserstoff oder Alkylgruppen mit 1 bis 5 Kohlenstoffatomen;
R₆ Wasserstoff, eine Hydroxylgruppe oder eine Alkylgruppe mit 1 bis 5 Kohlenstoffatomen; und
R₈ eine Acetoacetylgruppe bedeuten
mit einer Verbindung der allgemeinen Formel (IV): 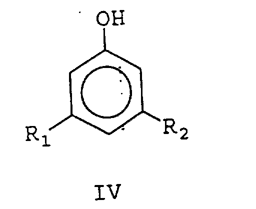 worin R₁ und R₂ Wasserstoff oder eine Hydroxylgruppe bedeuten, in Gegenwart von Phosphoroxychlorid oder von Chlorwasserstoffgas kondensiert wird, und gewünschtenfalls, falls Y und/oder R₅ Wasserstoff bedeuten, die Verbindung der Formel (I)
(i) mit einer α-Aminosäure in einen Ester überführt wird, oder
(ii) mit einem Aminoalkylhalogenid mit 1 bis 5 Kohlenstoffatomen zur Reaktion gebracht wird.

5. Eine Verbindung, wie sie in jedem der Ansprüche 1 bis 3 definiert ist, zur Verwendung als Arzneimittel.

6. Pharmazeutische Zusammensetzung, umfassend eine Verbindung, wie sie in jedem der Ansprüche 1 bis 3 definiert ist, und ein pharmazeutisch annehmbarer Träger.

7. Verwendung einer Verbindung, wie sie in jedem der Ansprüche 1 bis 3 definiert ist, für die Herstellung eines gegen HIV wirksamen Arzneimittels.

## Revendications

1. Composés de formule (I) : 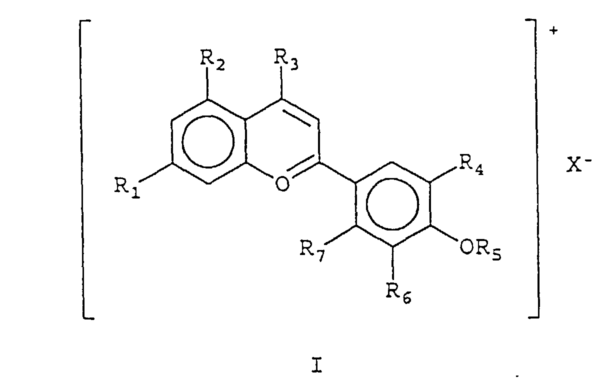 dans laquelle :
- X représente un anion non toxique ;
- R₁ et R₂ sont choisis indépendamment parmi l'hydrogène ou un groupe -OY dans lequel Y est l'hydrogène, un groupe α-amino-acyle ou un groupe α-amino-alkyle ayant 1 à 5 atomes de carbone ;
- R₅ représente l'hydrogène, un groupe α-amino-acyle ou un groupe amino-alkyle ayant 1 à 5 atomes de carbone ;
- R_{3,} R₄ et R₇ représentent indépendamment l'hydrogène ou un groupe alkyle ayant 1 à 5 atomes de carbone ; et
- R₆ représente l'hydrogène, un groupe hydroxy ou un groupe alkyle ayant 1 à 5 atomes de carbone.

2. Composé selon la revendication 1, dans lequel :
- X est un ion chlorure ; R₁ et R₂ représentent chacun un groupe -OY dans lequel Y est un groupe amino-alkyle ayant 1 à 5 atomes de carbone ou un groupe glycyle, alanyle, lysyle ou arginyle;
- R₅ est l'hydrogène ;
- R_{3,} R₄ et R₇ sont l'hydrogène ou un groupe méthyle ; et
- R₆ est l'hydrogène, un groupe hydroxy ou un groupe méthyle.

3. Composé selon la revendication 1, qui est le chlorure de 5,7,4'-trihydroxy-4,3',5'-triméthylflavilium.

4. Procédé pour la préparation de composés de formule (I) : 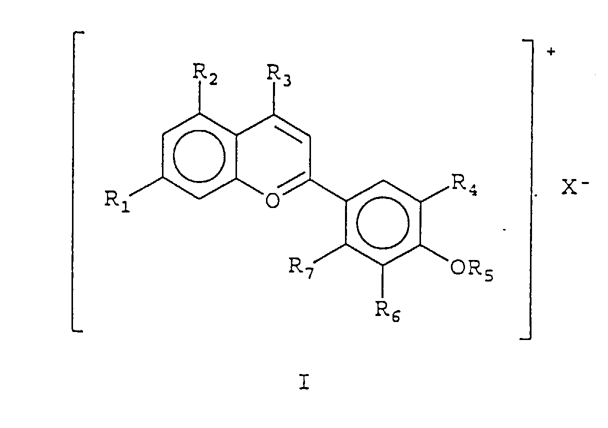 dans laquelle :
- X représente un anion non toxique ;
- R₁ et R₂ sont choisis indépendamment parmi l'hydrogène ou un groupe -OY dans lequel Y est l'hydrogène, un groupe α-amino-acyle ou un groupe α-amino-alkyle ayant 1 à 5 atomes de carbone ;
- R₅ représente l'hydrogène, un groupe α-amino-acyle ou un groupe amino-alkyle ayant 1 à 5 atomes de carbone ;
- R_{3,} R₄ et R₇ représentent indépendamment l'hydrogène ou un groupe alkyle ayant 1 à 5 atomes de carbone ; et
- R₆ représente l'hydrogène, un groupe hydroxy ou un groupe alkyle ayant 1 à 5 atomes de carbone,
lequel procédé est caractérisé en ce que :
(a) soit un dérivé du phénol de formule (II) 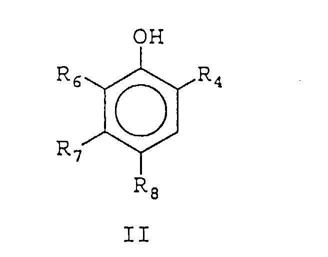 dans laquelle :
- R₄ et R₇ représentent l'hydrogène ou un groupe alkyle ayant 1 à 5 atomes de carbone ;
- R₆ représente l'hydrogène ou un groupe hydroxy ou un groupe alkyle ayant 1 à 5 atomes de carbone ; et
- R₈ représente l'hydrogène,
est condensé avec un dérivé de la coumarine de formule (III) : 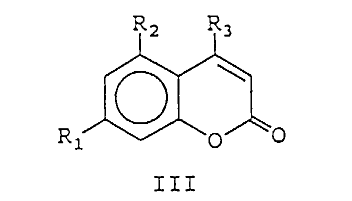 dans laquelle :
- R₁ et R₂ représentent l'hydrogène ou un groupe hydroxy ;
- R₃ représente l'hydrogène ou un groupe alkyle ayant 1 à 5 atomes de carbone ;
(b) soit un dérivé du phénol de formule (II) dans laquelle :
- R₄ et R₇ représentent l'hydrogène ou un groupe alkyle ayant 1 à 5 atomes de carbone ;
- R₆ représente l'hydrogène ou un groupe hydroxy ou un groupe alkyle ayant 1 à 5 atomes de carbone ; et
- R₈ représente un groupe acétoacétyle;
est condensé avec un dérivé de formule (IV) : 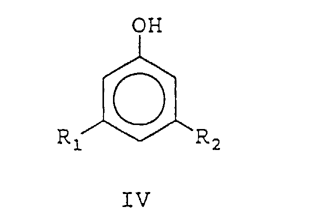 dans laquelle :
R₁ et R₂ représentent l'hydrogène ou un groupe hydroxy :
en présence d'oxychlorure de phosphore ou d'acide chlorhydrique gazeux, et si désiré, lorsque Y et/ou R₅ représentent l'hydrogène, le composé de formule (I) est :
- (i) transformé en un ester avec un acide α-amino ou
- (ii) mis en réaction avec un halogénure d'aminoalkyle ayant 1 à 5 atomes de carbone.

5. Composé selon l'une quelconque des revendications 1 à 3, en vue de l'emploi en tant que produit pharmaceutique.

6. Composition pharmaceutique comprenant un composé selon l'une quelconque des revendications 1 à 3 et un véhicule pharmaceutiquement acceptable.

7. Utilisation d'un composé selon l'une quelconque des revendications 1 à 3 pour la fabrication d'un médicament efficace contre le HIV.
